Europäisches Patentamt

European Patent Office

Office européen des brevets

(11) Numéro de publication: **0 278 807 B1**

## (12) FASCICULE DE BREVET EUROPEEN

(45) Date de publication de fascicule du brevet: **15.04.92**

(51) Int. Cl.⁵: **A61F 2/40**, A61F 2/30

(21) Numéro de dépôt: **88400079.5**

(22) Date de dépôt: **15.01.88**

(54) **Prothése d'épaule.**

(30) Priorité: **09.02.87 FR 8701524**

(43) Date de publication de la demande:
**17.08.88 Bulletin 88/33**

(45) Mention de la délivrance du brevet:
**15.04.92 Bulletin 92/16**

(84) Etats contractants désignés:
**AT BE CH DE ES GB GR IT LI LU NL SE**

(56) Documents cités:
EP-A- 0 127 503      EP-A- 0 149 527
CH-A- 543 881        DE-A- 3 216 111
FR-A- 2 541 890      GB-A- 2 162 753
US-A- 3 916 451      US-A- 3 978 528
US-A- 4 045 826      US-A- 4 106 130
US-A- 4 549 319      US-A- 4 550 450

(73) Titulaire: **Lannelongue, Jean**
**2, rue Ferdinand Dubreuil**
**F-37000 Tours(FR)**

(72) Inventeur: **Lannelongue, Jean**
**2, rue Ferdinand Dubreuil**
**F-37000 Tours(FR)**

(74) Mandataire: **Michardière, Bernard et al**
**C/O CABINET PEUSCET 68, rue d'Hauteville**
**F-75010 Paris(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention est relative à une prothèse d'épaule, du genre de celles qui comprennent une pièce humérale destinée à être fixée dans un humérus pour en remplacer au moins la tête et comprenant un élément tubulaire creux devant être fixé dans l'humérus sensiblement selon son axe longitudinal et une tige cylindrique dont l'extrémité supérieure porte une sphère coopérant avec une portée glénoïdienne pour former l'articulation de l'épaule et dont l'extrémité inférieure est disposée dans l'élément tubulaire précité, celui-ci et la tige pouvant coulisser l'un par rapport à l'autre, la portée glénoïdienne étant constituée par un pièce glénoïdienne qui comprend un élément d'ancrage destiné à être inséré dans une cavité pratiquée dans l'omoplate et un élément hémisphérique femelle, ce dernier coopérant avec la sphère mâle de la pièce humérale.

La pièce humérale doit être fixée dans l'extrémité supérieure de l'humérus pour en remplacer la tête et éventuellement le trochiter ; la pièce glénoïdienne, parfois appelée scapulaire, doit être introduite dans l'omoplate pour en remplacer la cavité glénoïde.

Comme c'est le cas pour toute prothèse articulaire, la fixation dans les os de chaque pièce constituant cette prothèse pose un problème important. En effet, cette fixation sera toujours de solidité limitée car il ne peut y avoir d'union totale microscopique entre le tissu vivant de l'os et le matériau inerte de la prothèse. Actuellement, il existe deux possibilités pour améliorer cette fixation : soit utiliser des prothèses à surface rugueuse de façon que l'os fabrique du tissu osseux moulant les différentes aspérités ; soit utiliser une substance plastique, qui s'infiltre dans les anfractuosités de l'os et solidarise ainsi la pièce à l'os; comme par exemple le méthacrylate de méthyle.

Malgré l'utilisation de l'une ou de l'autre de ces possibilités, la jonction os/prothèse reste toujours une zone de fragilité. Or, il est indispensable que cette jonction soit la plus solide possible afin de permettre les mouvements de l'articulation et éviter les douleurs provoquées en cas de mauvaise fixation. De plus, la prise de jeu entre la prothèse et l'os provoque une destruction de l'os et par suite entraîne un décrochement de la prothèse.

Dans le cas d'une prothèse d'épaule, constituée d'une pièce humérale et d'une pièce glénoïdienne, la pièce glénoïdienne est celle qui est la plus susceptible d'être désolidarisée de de l'omoplate : en effet, cet os est de très faible épaisseur, environ 1 cm, et de petite surface, de 4 à 6 cm2 environ. De plus, la pièce glénoïdienne est soumise à des poussées vers le haut provoquées par les muscles et les appuis de la main, et à des tractions vers le bas provoquées par le poids du bras lui-même ainsi que par les objets portés à la main. A l'état normal, il existe un groupe musculaire placé entre l'acromion, apophyse de l'omoplate, et la tête humérale qui sert de tampon et qui s'oppose au mouvement générant une pression vers le haut. Malheureusement, ce groupe musculaire s'use et c'est justement, quand il est usé, que doit être envisagée la mise en place d'une prothèse, qui ne peut donc pas utiliser ce groupe pour éviter les mouvements d'ascension.

On a proposé dans DE-A-3 216 111 une prothèse d'épaule comprenant une pièce humérale destinée à être fixée dans un humérus pour en remplacer au moins la tête qui coopère avec une portée destinée à remplacer l'articulation de l'humérus sur l'omoplate. La pièce humérale comprend un logement tubulaire devant être introduit et fixé dans l'humérus sensiblement selon l'axe de l'humérus, et une tige cylindrique ayant une section droite sensiblement égale à celle du logement et coulissant dans le logement tubulaire.

Un des buts de la présente invention est de fournir une prothèse d'épaule comprenant une pièce humérale et éventuellement une pièce glénoïdienne, du genre défini précédemment, dont la fixation dans l'omoplate soit de plus longue durée.

Un autre but de la présente invention est de fournir une telle prothèse qui soit stable, c'est-à-dire que les pièces la constituant ne soient pas sujettes à se déplacer les unes par rapport aux autres, comme dans le cas d'une luxation.

Ces buts, ainsi que d'autres qui apparaîtront par la suite, sont atteints par une prothèse d'épaule du genre défini précédemment, caractérisée par le fait que l'élément d'ancrage comporte, d'une part, à sa partie supérieure, un aileron qui fait avec la verticale un angle de 30° environ vers l'avant, qui est situé en retrait par rapport à la face latérale de l'élément d'ancrage et qui est destiné à être introduit dans la base d'implantation de l'apophyse coracoïde de l'omoplate et d'autre part, à sa partie inférieure, un orifice pour le passage d'un moyen de fixation dans le pilier de l'omoplate.

Dans la présente description et dans les revendications annexées, les qualifications "supérieur", "inférieur", "horizontal", "vertical", "avant" et "arrière" sont utilisées en supposant un sujet en position verticale, tête en haut, ayant les bras dans la position qu'ils prennent sous le seul effet de la pesanteur, observé de face.

Avantageusement, la paroi externe de l'élément tubulaire est évasée à son extrémité supérieure en un tronc de cône, dont les bases sont sensiblement perpendiculaires à l'axe de cet élément tubulaire, la base supérieure affleurant la section transversale faite pour supprimer la tête de l'humérus ; cette base supérieure du tronc de cône est, de préférence, prolongée, en direction de la face interne de l'humérus, par un bec plat ; l'extrémité inférieure de l'élément tubulaire est de forme arrondie ; la section droite de la tige et celle du logement intérieur de l'élément tubulaire sont circulaires.

Afin d'améliorer le maintien de cette pièce humérale dans l'humérus, il est avantageux que la surface extérieure de cet élément tubulaire comporte des sillons horizontaux vers l'extrémité supérieure de celui-ci ainsi que des sillons verticaux.

De préférence, l'élément tubulaire a une longueur comprise entre 8 et 10 cm, une épaisseur de paroi comprise entre 0,15 et 0,25 cm, un diamètre externe compris entre 10 et 14 mm, le tronc de cône ayant une hauteur de 2 à 2,5 cm et la base supérieure de celui-ci un diamètre de 20 à 30 mm ; le logement intérieur a une profondeur comprise entre 7 et 9 cm.

La tige, du côté de son extrémité supérieure, comporte un disque perpendiculaire à son axe, disposé à une certaine distance de la sphère. La distance entre l'extrémité inférieure de la tige et la face inférieure du disque est au plus égale à la profondeur du logement intérieur de l'élément tubulaire. La distance entre la face supérieure du disque, tournée vers la sphère, et la base de cette sphère, est comprise avantageusement entre 12 et 15 mm ; le diamètre de la sphère est compris entre 10 et 15 mm environ quand on utilise une pièce glénoïdienne et de 35 à 50 mm quand on utilise la glène de l'omoplate.

Selon un mode de réalisation avantageux, l'élément d'ancrage et l'élément hémisphérique de la pièce glénoïdienne sont deux éléments distincts réunis par un moyen de liaison.

Selon un mode de réalisation préféré, l'élément d'ancrage est constitué par une masse, dont les sections horizontales sont des triangles sensiblement isocèles ; la partie inférieure de ladite masse comporte une face externe sensiblement plane qui affleure l'os au niveau de la glène et porte en saillie un plot parallélépipédique muni de moyens de retenue coopérant avec le moyen de liaison, une ailette étant prévue à la base de ladite face externe pour se trouver en vis-à-vis du pilier de l'omoplate, ladite ailette comportant l'orifice pour le passage du moyen de fixation s'enfonçant dans ledit pilier, la partie supérieure de ladite masse formant la liaison entre ladite partie inférieure et l'aileron, la largeur de ladite masse dans sa face externe, mesurée horizontalement, étant maximale au niveau du plot et se réduisant progressivement vers l'ailette et l'aileron, un rétrécissement étant néanmoins prévu entre les parties supérieure et inférieure, l'aileron étant situé en retrait par rapport à la face externe et étant relié à elle par un plan oblique par rapport à la face externe. Par exemple le moyen coopérant avec le moyen de liaison peut être un tunnel traversant le plot de part en part, le moyen de liaison étant alors une goupille.

Avantageusement, l'élément hémisphérique comporte une cupule hémisphérique dans laquelle est logée la sphère de la pièce humérale, et une embase pouvant coiffer le plot de l'élément d'ancrage. Cette cupule hémisphérique est légèrement rétentive par rapport à la sphère de la pièce humérale. Dans certains cas, l'élément hémisphérique peut comporter une plaquette, qui prend appui sur la face interne de l'acromion de l'omoplate dans laquelle la pièce glénoïdienne est fixée.

L'élément tubulaire et l'élément hémisphérique sont avantageusement réalisés en un polyéthylène haute densité, tandis que la tige ainsi que l'élément d'ancrage sont métalliques.

La description qui va suivre et qui ne présente aucun caractère limitatif, doit être lue en regard des figures annexées, parmi lesquelles :

- la figure 1 représente une vue ventrale, c'est-à-dire une vue avant, d'une omoplate et d'un humérus, en l'occurence de l'omoplate et de l'humérus droits ;
- la figure 2 est une vue latérale de la zone d'articulation humérale de l'omoplate de la figure 1 ;
- la figure 3 est une vue en coupe de l'omoplate de la figure 1, selon la ligne III-III de la figure 1 ;
- la figure 4 est une prothèse d'épaule selon l'invention une fois mise en place, l'humérus étant coupé ;
- la figure 5 est une vue en éclaté et en coupe de la pièce humérale de la prothèse d'épaule de la figure 4;
- la figure 6 est une vue de dessus selon la ligne VI-VI de la figure 5 ;

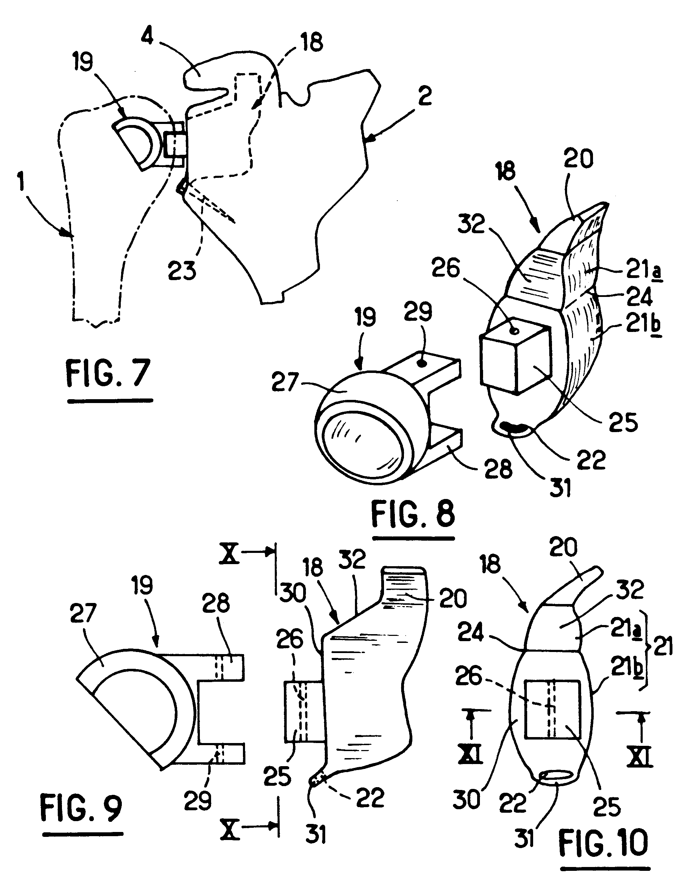

- la figure 7 est une vue correspondant à la figure 1, la partie glénoïdienne de la prothèse d'épaule étant implantée dans l'omoplate, l'humérus initial étant représenté en traits mixtes ;
- la figure 8 est une vue en perspective éclatée des deux éléments de la pièce glénoïdienne, selon un mode de réalisation de l'invention ;
- la figure 9 est une vue latérale des éléments représentés à la figure 8 ;
- la figure 10 est une vue selon la ligne X-X de la figure 9 ;
- la figure 11 est une coupe selon la ligne XI-XI de la figure 10 ;
- la figure 12 représente une variante de réalisation de l'élément tubulaire de la pièce humérale, objet de la figure 5 ; et

- la figure 13 représente une variante de réalisation de la tige de la pièce humérale, objet de la figure 5.

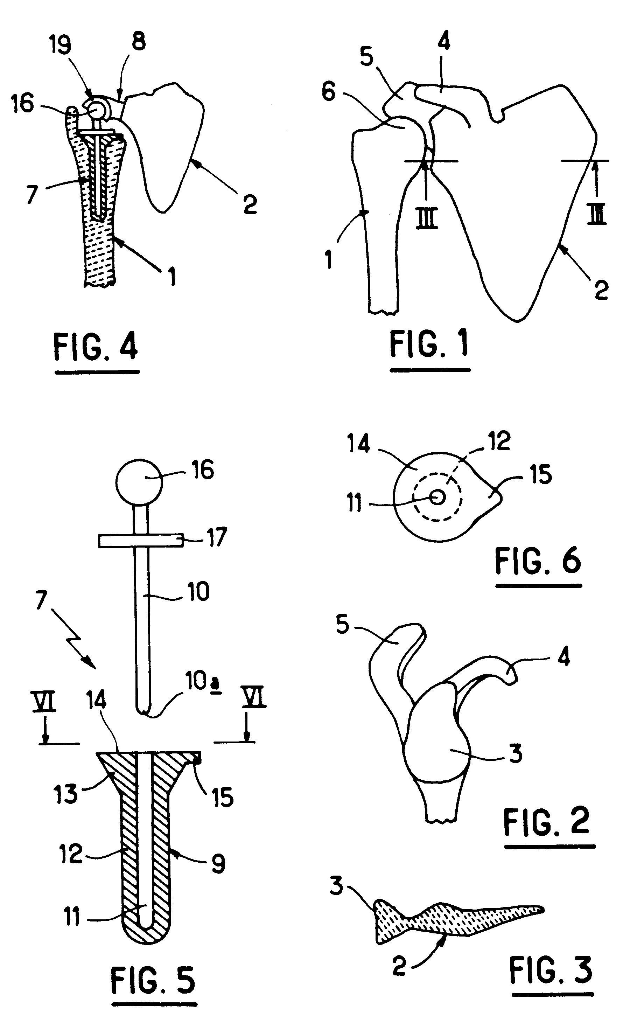

Comme on peut le voir sur les figures 1 à 3, une épaule est l'articulation d'un humérus 1 avec la ceinture scapulaire et en particulier avec l'omoplate 2 de cette ceinture.

L'omoplate 2 est un os de forme sensiblement triangulaire, dont un des sommets est dirigé vers le bas. Cet os est relativement plat ainsi qu'on peut s'en rendre compte sur la figure 3 : son épaisseur est d'environ 1 cm, ce qui est source de beaucoup de problèmes pour introduire dans cette épaisseur un élément de prothèse. Il comporte en fait deux renflements l'un dénommé pilier, situé sensiblement le long de son côté en regard de l'humérus 1, et l'autre partant de la cavité glénoïde 3 et correspondant à l'implantation de l'apophyse coracoïde 4, qui est située sur la face avant de l'omoplate. La cavité glénoïde 3 est la cavité que présente l'omoplate 2 en regard de la tête 6 de l'humérus 1. Cette cavité glénoïde 3 a la forme générale d'une poire, dont le sommet serait dirigé vers l'apophyse coracoïde 4. Sur la face dorsale, ou face arrière de cette omoplate, et sensiblement en regard de l'implantation de l'apophyse coracoïde 4 est situé l'acromion 5.

La prothèse d'épaule selon la présente invention, comme représentée à la figure 4, comprend une pièce humérale 7 et une pièce glénoïdienne 8 : la pièce humérale 7 est implantée dans l'os de l'humérus 1, selon l'axe longitudinal de cet os ; la pièce glénoïdienne 8 est implantée dans la cavité glénoïde 3 et vient recouvrir le sommet de la pièce humérale 7, ainsi que cela sera décrit plus loin.

La pièce humérale 7 comprend un élément tubulaire 9, qui est creux et qui est implanté dans l'os de l'humérus, et une tige pleine 10 sensiblement cylindrique. L'élément tubulaire 9 comprend donc un logement ou canal longitudinal 11 ouvert à sa partie supérieure et fermé à sa partie inférieure. La paroi externe 12 de l'élément tubulaire est évasée à son extrémité supérieure selon un tronc de cône 13, dont la base supérieure est perpendiculaire à l'axe de l'élément tubulaire 9 et affleure la section pratiquée pour enlever la tête de l'humérus quand on veut mettre en place la prothèse (voir figure 1). En direction de la face interne de l'humérus 1, la base supérieure 14 du tronc de cône 13 est prolongée par un bec plat 15. La tige 10 et le canal 11 ont des sections circulaires égales au jeu près.

Les dimensions de l'élément tubulaire 9 sont fonction de celles de l'humérus 1. On considère qu'il suffit d'utiliser l'un des trois modèles regroupés dans le tableau I pour couvrir tous les types d'humérus.

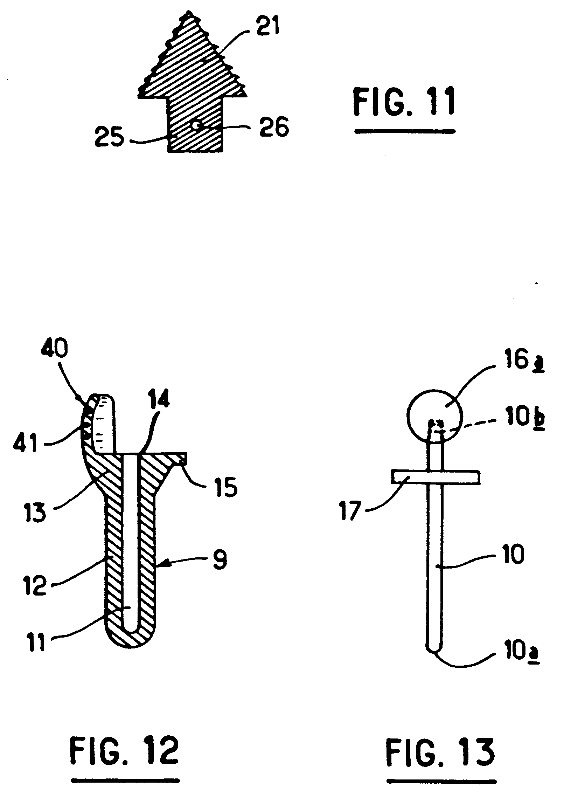

Selon une variante, représentée à la figure 12, l'élément tubulaire 9 peut comporter un bouclier 40 en arc de cercle disposé à la périphérie de la base supérieure 14 du tronc de cône 13 et à l'opposé du bec plat 15. Ce bouclier doit être notamment présent pour rattacher les muscles, par ligature au moyen de fils passant dans des tunnels 41, au nombre de 2 ou 3, lorsque la partie correspondante de l'humérus ne peut être conservée. Les tunnels 41 sont percés dans l'épaisseur du bouclier 40 perpendiculairement à son rayon de courbure. Par exemple, ce bouclier a une hauteur de 3 cm et une largeur de 3 cm. Quant à sa courbure, elle doit être identique à celle du disque 17 de la tige 10 décrite ci-après.

L'autre partie de la pièce humérale, la tige 10m comporte à son extrémité supérieure une sphère 16, tandis que son extrémité inférieure 10a est disposée dans le canal 11 de l'élément tubulaire 9 : ce dernier et la tige 10 peuvent coulisser l'un par rapport à l'autre.

A une distance de son extrémité inférieure 10a égale à la longueur du canal intérieur de l'élément tubulaire 9, la tige 10 porte un disque 17, qui est perpendiculaire à l'axe de ladite tige. La face inférieure de ce disque 17 peut venir en contact avec la base supérieure 14 du tronc de cône 13. L'épaisseur du disque 17, qui est normalement de 2 mm, peut varier entre 3 mm et 2 cm façon à compenser un raccourcissement de l'humérus nécessarie pour l'implantation de la prothèse selon l'invention. La distance entre la face supérieure du disque 17, c'est-à-dire celle tournée vers la sphère 16, et la base de cette sphère 16 est comprise entre 12 et 15 mm en fonction des modèles de pièces humérales, comme indiqué dans le tableau I ci-après. Quant au diamètre de la sphère 16, il est également fonction du modèle de pièces humérales : il peut varier de 15 à 30 mm.

Une sphère plus grosse peut être également prévue, car dans certains cas l'omoplate n'est pas usée. Il suffit, dans ce cas, de remplacer la tête 6 de l'humérus 1 : il n'y a donc pas de pièce fixée dans l'omoplate, et en conséquence la sphère 16 doit occuper tout le volume normal de la tête 6 de l'humérus 1. Le diamètre d'une telle sphère devra donc se rapprocher de celui de la tête humérale normale : de ce fait il variera entre 35 et 50 mm. Cette sphère de plus grand diamètre peut également être réalisée à partir d'une sphère de petit diamètre, telle que précédemment décrite, sur laquelle est disposée une cupule rétentive, qui n'est pas fixée à l'omoplate 2. Cet ensemble permet de répartir les mouvements en partie entre la sphère et la cupule, d'une part, et la cupule et l'omoplate, d'autre part.

EP 0 278 807 B1

## TABLEAU I

|  | Grande taille | Taille moyenne | Petite taille |
|---|---|---|---|
| Elément tubulaire (9) |  |  |  |
| . Longueur extérieure | 10 cm | 8 cm | 6 cm |
| . Longueur du canal (11) | 9 cm | 7 cm | 7 cm |
| . Diamètre extérieur | 12 mm | 10 mm | 10 mm |
| . Diamètre du canal (11) | 8 mm | 6 mm | 6 mm |
| Hauteur du tronc de cône (13) | 2 cm | 2 cm | 2 cm |
| Disque (17) diamètre | 30 mm | 25 mm | 20 mm |
|             hauteur | 2 mm | 2 mm | 2 mm |
| Tige (10) |  |  |  |
| . Longueur entre extrémité inférieure (10a) et le disque (17) | 9 cm | 7 cm | 7 cm |
| . Diamètre | 8 mm | 6 mm | 6 mm |
| Sphère (16) |  |  |  |
| . Diamètre | 30 mm | 25 mm | 15 mm |

Selon une variante de réalisation représentée à la figure 13, la tige 10 comporte à son extrémité supérieure une sphère 16a qui est emmanchée à force sur l'extrémité 10b de la tige qui est conformée en tronc de cône, dit cône Morse. Dans ce cas, la tige 10 peut être identique pour tous les modèles de prothèse : seule la sphère 16a étant spécifique. Dans cette variante, les dimensions des éléments de la pièce humérale 7 sont regroupées dans le tableau II ci-après.

5

## TABLEAU II

|  | Grande taille | Taille moyenne | Petite taille |
|---|---|---|---|
| Elément tubulaire (9) | | | |
| . Longueur extérieure | 10 cm | 8 cm | 8 cm |
| . Longueur du canal (11) | 7 cm | 7 cm | 7 cm |
| . Diamètre extérieur | 12 mm | 10 mm | 10 mm |
| . Diamètre du canal (11) | 6 mm | 6 mm | 6 mm |
| Hauteur du tronc de cône (13) | 2 cm | 2 cm | 2 cm |
| Disque (17) diamètre | 20 mm | 20 mm | 20 mm |
| hauteur | 2 mm | 2 mm | 2 mm |
| Tige (10) | | | |
| . Longueur entre extrémité inférieure (10a) et le disque (17) | 9 cm | 7 cm | 7 cm |
| . Diamètre | 6 mm | 6 mm | 6 mm |
| Sphère. (16) | | | |
| . Diamètre | 30 mm | 25 mm | 15 mm |

Ainsi qu'il a été dit précédemment, la prothèse d'épaule selon le mode de réalisation de la présente invention comprend, d'une part, la pièce humérale 7 décrite ci-dessus et, d'autre part, la pièce glénoïdienne 8. Cette dernière est, selon le présent exemple de réalisation, constituée de deux éléments : un élément d'ancrage 18, qui est destiné à être introduit, partiellement, dans l'omoplate au niveau de la cavité glénoïdienne 3, et un élément hémisphérique 19, qui coopère avec la sphère 16 de la pièce humérale 7. L'élément d'ancrage 18 et l'élément hémisphérique 19 sont réunis par une goupille (non représentée).

L'élément d'ancrage 18 comporte, d'une part, un aileron plat 20 et, d'autre part, une masse 21 ayant en coupe verticale sensiblement la forme d'une poire, tout commé la cavité glénoïde 3. Cette masse 21, dont les sections horizontales sont des triangles isocèles, et dont les parois destinées à pénétrer dans l'omoplate comportent des stries horizontales, comme représenté à la figure 11, comprend une partie supérieure 21a surmontée par l'aileron 20 et une partie inférieure 21b comportant à sa base une ailette 31 munie d'un orifice 22 pour le passage d'une vis 23, constituant un moyen de fixation dans le pilier de l'omoplate 2. Entre les parties supérieure 21a et inférieure 21b existe un rétrécissement 24 de chaque côté de la masse 21.

L'élément d'ancrage 18 comprend également un plot 25 disposé sur la face externe 30 de la partie inférieure 21b de la masse 21 : ce plot est de forme parallélépipédique et comporte un tunnel vertical 26 le traversant de part en part pour le passage de la goupille permettant de fixer l'élément hémisphérique 19 sur l'élément d'ancrage 18. La zone la plus large de la face externe 30 est située au niveau du plot 25 et la masse 21 a une largeur, mesurée d'avant en arrière, qui diminue progressivement vers l'ailette 31 et vers l'aileron 20. L'aileron 20 est situé en retrait par rapport à la face externe 30 et il se raccorde à elle par un plan oblique 32. La bordure inférieure de la partie 21b est arrondie depuis la face externe 30 vers la zone centrale de l'omoplate pour faciliter l'introduction de la prothèse dans la cavité qui lui est destinée.

L'élément hémisphèrique 19 comporte une cupule 27 disposée sur une embase 28 en forme de U, qui vient coiffer le plot 25 de l'élément d'ancrage 18. L'embase 28 comprend également un passage 29, qui vient dans le prolongement du tunnel 26 pour permettre le logement de la goupille assurant la réunion des deux éléments de la pièce glénoïdienne 8.

La cupule hémisphérique 27, lorsque la prothèse d'épaule est mise en place, vient coiffer la tête 16 de la pièce humérale 7 et exerce sur cette tête une faible action rétentive, puisque son volume est légèrement supérieur à celui d'une demi-sphère de même diamètre. Ce caractère légèrement rétentif assure une stabilité dans les mouvements, tout en permettant un déboîtement des deux pièces de la prothèse évitant par là-même une fracture de l'omoplate ou un décrochement de ces pièces au niveau de l'os.

Lorsque l'on introduit la pièce glénoïdienne 8 dans une omoplate 2, l'aileron 20 vient se situer à la base de l'implantation de l'apophyse caracoïde 4 tandis que l'orifice 22 permet d'introduire la vis 23 dans le pilier de cette omoplate 2. On profite ainsi des parties les plus épaisses de l'omoplate. De ce fait, l'aileron 20 fait avec la verticale un angle de 30° environ vers l'avant.

Dans le tableau III ci-dessous, sont regroupées les caractéristiques dimensionnelles de la pièce glénoïdienne 8 selon les trois modèles les plus courants qui coopèrent avec les modèles correspondants de la pièce humérale 7.

## TABLEAU III

|  | Grand modèle | Modèle moyen | Petit modèle |
|---|---|---|---|
| Elément d'ancrage (18) |  |  |  |
| . Hauteur | 35 mm | 30 mm | 30 mm |
| . Largeur partie supérieure (21a) | 15 mm | 13 mm | 8 mm |
| . Largeur partie inférieure (21b) | 20 mm | 17 mm | 12 mm |
| . Longueur du côté du triangle coupe de la masse (21) | 20 mm | 15 mm | 10 mm |
| Aileron (20) |  |  |  |
| . Longueur | 10 mm | 10 mm | 10 mm |
| . Largeur | 10 mm | 10 mm | 10 mm |
| . Epaisseur | 2 mm (a) | 2 mm (a) | 2 mm (a) |
|  | 4 mm (b) | 4 mm (b) | 2 mm (b) |
| Plot (25) |  |  |  |
| . Hauteur | 15 mm | 15 mm | 15 mm |
| . Largeur | 10 mm | 10 mm | 10 mm |
| . Epaisseur | 15 mm | 15 mm | 15 mm |
| (a) métal (b) matière plastique |  |  |  |

L'élément tubulaire 9 de la pièce humérale 7 ainsi que l'élément hémisphérique 19 de la pièce glénoïdienne 8 sont réalisés en un polyéthylène haute densité ; la tige 10 de la pièce humérale 7 et l'élément d'ancrage 18 de la pièce glénoïdienne 8 sont en acier inoxydable.

La prothèse d'épaule, qui vient d'être décrite, permet donc, d'une part, de maintenir une stabilité entre les deux pièces la composant tout en permettant une grande mobilité ainsi qu'il a été dit et, d'autre part, de diminuer les forces agissant sur la fixation des différentes pièces dans les os correspondants en permettant un mouvement de rotation et un mouvement longitudinal entre l'élément huméral 9, qui est fixé dans l'humérus 1, et la tige 10 portant la sphère 16. En effet, la tige 10 est mobile à l'intérieur du canal 11 de l'élément huméral 9, qui, lui, est fixé dans l'os de l'humérus 1.

Ceci présente notamment comme avantage de pouvoir prendre en charge les mouvements de rotation et éviter qu'ils se produisent au niveau de la sphère 16. Les mouements de rotation sont définis en anatomie comme étant des mouvements qui se produisent autour de l'axe longitudinal du bras. Ces mouvements sont très utilisés dans la vie courante et ce sont eux qui favorisent les luxations de l'articulation normale. Ce sont également eux qui sont commandés et freinés par les muscles dits de la coiffe, qui sont très souvent usés au niveau de l'épaule.

Cette prothèse présente également comme avantage de supprimer les sollicitations sur les pièces de haut en bas. En effet, le poids du bras et de la main tend à tirer vers le sol la pièce humérale, et par son intermédiaire, la pièce fixée dans l'omoplate lorsqu'il existe une stabilité rétentive : ces sollicitations sont supprimées, ou tout au moins fortement absorbées, par la possibilité de coulissement de la tige 10 dans l'élément huméral 9.

Si les muscles du tampon ont complètement disparu, on fixe sur l'élément hémisphérique 19 de la pièce glénoïdienne 8 une plaquette, qui prend appui sur l'acromion 5 (non représentée).

**Revendications**

1. Prothèse d'épaule comprenant une pièce humérale (7) destinée a être fixée dans un humérus (1) pour en remplacer au moins la tête et comprenant un élément tubulaire (9) creux devant être fixé dans l'humérus (1) sensiblement selon son axe longitudinal et une tige (10) cylindrique dont l'extrémité supérieure porte une sphère (16) coopérant avec une portée glénoïdienne pour former l'articulation de l'épaule et dont l'extrémité inférieure est disposée dans l'élément tubulaire (9) précité, celui-ci et la tige (10) pouvant coulisser l'un par rapport à l'autre, la tige (10) cylindrique ayant une section droite sensiblement égale à celle du logement (11) qui constitue le creux à l'intérieur de l'élément tubulaire (9), la portée glénoïdienne étant constituée par une pièce glénoïdienne (8) qui comprend un élément d'ancrage destiné à être inséré dans une cavité pratiquée dans l'omoplate (2) et un élément hémisphérique (19) femelle, ce dernier coopérant avec la sphère mâle (16) de la pièce humérale (7) caractérisée par le fait que l'élément d'ancrage (18) comporte, d'une part, à sa partie supérieure, un aileron (20) qui fait avec la verticale un angle de 30° environ vers l'avant, qui est situé en retrait par rapport à la face latérale de l'élément d'ancrage et qui est destiné à être introduit dans la base d'implantation de l'apophyse coracoïde (4) de l'omoplate (2) et d'autre part, à sa partie inférieure un orifice (22) pour le passage d'un moyen de fixation (23) dans le pilier de l'omoplate (2).

2. Prothèse d'épaule selon la revendication 1, caractérisée par le fait que la paroi externe (12) de l'élément tubulaire (7) est évasée à son extrémité supérieure en un tronc de cône (13), dont les bases sont sensiblement perpendiculaires à l'axe dudit élément tubulaire (7), la base la base supérieure (14) affleurant la section transversale faite pour supprimer la tête (6) de l'humérus (1).

3. Prothèse d'épaule selon la revendication 2, caractérisée par le fait que la base supérieure (14) du tronc de cone (13) est prolongée, en direction de la face interne de l'humérus (1) par un bec (15) plat.

4. Prothèse d'épaule selon l'une des revendications 1 à 3, caractérisée par le fait que l'extrémité inférieure de l'élément tubulaire (9) est de forme arrondie.

5. Prothèse d'épaule selon l'une des revendications 1 à 4, caractérisée par le fait que la section droite de la tige (10) et celle du logement intérieur (11) de l'élément tubulaire (9) sont circulaires.

6. Prothèse d'épaule selon l'une des revendications 1 à 5, caractérisée par le fait que la surface extérieure de l'élément tubulaire (9) comporte des sillons horizontaux vers l'extrémité supérieure de celui-ci, ainsi que des sillons verticaux.

7. Prothèse d'épaule selon l'une des revendications 1 à 6, caractérisée par le fait que l'élément tubulaire (7) a une longueur comprise entre 8 et 10 cm, une épaisseur de paroi (12) comprise entre 0,15 et 0,25 cm, un diamètre externe compris entre 10 et 12 mm, que le tronc de cône (13) a une hauteur de 2 à 2,5 cm et la base supérieure (14) de celui-ci un diamètre de 20 à 30 mm, et que le logement intérieur (11) a une profondeur comprise entre 7 et 9 cm.

**8.** Prothèse d'épaule selon l'une des revendications 1 à 7, caractérisée par le fait que la tige (10) du côté de son extrémité supérieure, comporte un disque (17) perpendiculaire à son axe, disposé à une certaine distance de la sphère (16).

**9.** Prothèse d'épaule selon la revendication 8, caractérisée par le fait que la distance entre l'extrémité inférieure (10a) de la tige (10) et la face inférieure du disque (17) est au plus égale à la profondeur du logement intérieur (11) de l'élément tubulaire (9).

**10.** Prothèse selon l'une des revendications 8 ou 9, caractérisée par le fait que la distance entre la face supérieure du disque (17), tournée vers la sphère (16), et la base de ladite sphère (16) est comprise entre 12 et 15 mm.

**11.** Prothèse d'épaule selon l'une des revendications 1 à 10, caractérisée par le fait que le diamètre de la sphère (16) est compris entre 10 et 15 mm environ.

**12.** Prothèse d'épaule selon l'une des revendications 1 à 10, caractérisée par le fait que le diamètre de la sphère (16) est compris entre 35 et 50 mm.

**13.** Prothèse d'épaule selon la revendication 1, caractérisée par le fait que l'élément d'ancrage (18) et l'élément hémisphérique (19) sont deux éléments distincts réunis par un moyen de liaison.

**14.** Prothèse d'épaule selon la revendication 1, caractérisée par le fait que le moyen de fixation est une vis (23).

**15.** Prothèse d'épaule selon l'une des revendications 1, 13 ou 14, caractérisée par le fait que l'élément d'ancrage (18) est constitué par une masse (21), dont les sections horizontales sont des triangles sensiblement isocèles.

**16.** Prothèse d'épaule selon la revendication 15, caractérisée par le fait que la partie inférieure (21b) de ladite masse (21) comporte une face externe (30) sensiblement plane qui affleure l'os au niveau de la glène et porte en saillie un plot (25) parallélépipédique muni de moyens de retenue (26) coopérant avec le moyen de liaison, une ailette (31) étant prévue à la base de ladite face externe (30) pour se trouver en vis-à-vis du pilier de l'omoplate (2), ladite ailette (31) comportant l'orifice (22) pour le passage du moyen de fixation (23) s'enfonçant dans ledit pilier, la partie supérieure (21a) de ladite masse(21) formant la liaison entre ladite partie inférieure (21b) et l'aileron (20), la largeur de ladite masse (21) dans sa face externe, mesurée horizontalement, étant maximale au niveau du plot (25) et se réduisant progressivement vers l'ailette (31) et l'aileron (20), un rétrécissement (24) étant néanmoins prévu entre les parties supérieures (21a) et inférieure (21b), l'aileron (20) étant situé en retrait par rapport à la face externe (30) et étant relié à elle par un plan oblique (32) par rapport à ladite face externe.

**17.** Prothèse d'épaule selon la revendication 16, caractérisée par le fait que le moyen coopérant avec le moyen de liaison est un tunnel vertical (26) traversant le plot (25) de part en part, le moyen de liaison étant une goupille.

**18.** Prothèse d'épaule selon la revendication 1, caractérisée par le fait que l'élément hémisphérique (19) comporte une cupule hémisphérique (27), dans laquelle est logée la sphère (16) de la pièce humérale (7), et une embase (28) pouvant coiffer le plot (25) de l'élément d'ancrage (18).

**19.** Prothèse d'épaule selon la revendication 18, caractérisée par le fait que la cupule hémisphérique (27) est légèrement rétentive par rapport à la sphère (16) de la pièce humérale (7).

**20.** Prothèse d'épaule selon l'une des revendications 1 à 18, caractérisée par le fait que l'élément hémisphérique (19) comporte une plaquette, qui prend appui sur la face interne de l'acromion (5) de l'omoplate (2), dans laquelle la pièce glénoïdienne (8) est fixée.

**Claims**

1. Shoulder prosthesis comprising a humeral part (7) adapted to be fixed in a humerus (1) in order to replace at least the head thereof and comprising a hollow tubular element (9) to be fixed in the humerus (1) substantially along its longitudinal axis and a cylindrical rod (10), the upper end of which is provided with a ball (16) cooperating with a glenoid bearing surface in order to form the shoulder joint and the lower end of which is disposed in the aforesaid tubular element (9), the latter and the rod (10) being slidable relative to one another, the cylindrical rod (10) having a cross section substantially equal to that of the housing (11) which forms the hollow in the interior of the tubular element (9), the glenoid bearing surface being formed by a glenoid part (8) which comprises an anchoring element adapted to be inserted into a cavity formed in the shoulder blade (2) and a female hemispherical element (19), the latter cooperating with the male ball (16) of the humeral part (7), characterised in that the anchoring element (18) comprises, on the one hand, in its upper part, a fin (20) forming an angle of approximately 30° with the vertical towards the front, recessed with respect to the lateral face of the anchoring element and adapted to be inserted into the implantation base of the coracoid process (4) of the shoulder blade (2) and, on the other hand, in its lower part, an orifice (22) for the passage of a fixing means (23) into the spine of the shoulder blade (2).

2. Shoulder prosthesis according to claim 1, characterised in that the outer wall (12) of the tubular element (7) is widened at its upper end into a truncated cone (13), the bases of which are substantially perpendicular to the axis of the said tubular element (7), the upper base (14) being flush with the transverse cut made to remove the head (6) of the humerus (1).

3. Shoulder prosthesis according to claim 2, characterised in that the upper base (14) of the truncated cone (13) is extended in the direction of the inner face of the humerus (1) by a flat rostrum (15).

4. Shoulder prosthesis according to one of claims 1 to 3, characterised in that the lower end of the tubular element (9) is rounded.

5. Shoulder prosthesis according to one of claims 1 to 4, characterised in that the cross sections of the rod (10) and the inner housing (11) of the tubular element (9) are circular.

6. Shoulder prosthesis according to one of claims 1 to 5, characterised in that the outer surface of the tubular element (9) has horizontal grooves towards its upper end, and also vertical grooves.

7. Shoulder prosthesis according to one of claims 1 to 6, characterised in that the tubular element (7) has a length of between 8 and 10 cm, a wall (12) thickness of between 0.15 and 0.25 cm and an outer diameter of between 10 and 12 mm, that the truncated cone (13) has a height of 2 to 2.5 cm and the upper base (14) of the latter has a diameter of 20 to 30 mm, and that the inner housing (11) has a depth of between 7 and 9 cm.

8. Shoulder prosthesis according to one of claims 1 to 7, characterised in that the rod (10), on the side of its upper end, has a disc (17) perpendicular to its axis, disposed at a certain distance from the ball (16).

9. Shoulder prosthesis according to claim 8, characterised in that the distance between the lower end (10a) of the rod (10) and the inner face of the disc (17) is at least equal to the depth of the inner housing (11) of the tubular element (9).

10. Prosthesis according to one of claims 8 and 9, characterised in that the distance between the upper face of the disc (17) directed towards the ball (16) and the base of the said ball (16) is between 12 and 15 mm.

11. Shoulder prosthesis according to one of claims 1 to 10, characterised in that the diameter of the ball (16) is between approximately 10 and 15 mm.

12. Shoulder prosthesis according to one of claims 1 to 10, characterised in that the diameter of the ball

EP 0 278 807 B1

(16) is between 35 and 50 mm.

13. Shoulder prosthesis according to claim 1, characterised in that the anchoring element (18) and the hemispherical element (19) are two separate elements joined together by a connecting means.

14. Shoulder prosthesis according to claim 1, characterised in that the fixing means is a screw (23).

15. Shoulder prosthesis according to one of claims 1, 13 and 14, characterised in that the anchoring element (18) consists of a mass (21), the horizontal sections of which are substantially isosceles triangles.

16. Shoulder prosthesis according to claim 15, characterised in that the lower part (21b) of the said mass (21) has a substantially flat outer face (30) which is flush with the bone at the socket and has a projecting parallelepipedal stud (25) provided with retaining means (26) cooperating with the connecting means, a wing (31) being provided at the base of the said outer face (30) so as to be situated opposite the spine of the shoulder blade (2), the said wing (31) having the orifice (22) for the passage of the fixing means (23) penetrating into the said spine, the upper part (21a) of the said mass (21) forming the connection between the said lower part (21b) and the fin (20), the width of the said mass (21) in its outer face, measured horizontally, being at a maximum at the stud (25) and gradually diminishing towards the wing (31) and the fin (20), a narrowed portion (24) still being provided between the upper and lower parts (21a) and (21b), the fin (20) being recessed with respect to the outer face (30) and being connected thereto by a plane (32) which is oblique with respect to the said outer face.

17. Shoulder prosthesis according to claim 16, characterised in that the means cooperating with the connecting means is a vertical tunnel (26) completely traversing the stud (25), the connecting means being a pin.

18. Shoulder prosthesis according to claim 1, characterised in that the hemispherical element (19) has a hemispherical cup (27), in which the ball (16) of the humeral part (7) is housed, and a flange (28) which can cover the stud (25) of the anchoring element (18).

19. Shoulder prosthesis according to claim 18, characterised in that the hemispherical cup (27) is slightly retentive with respect to the ball (16) of the humeral part (7).

20. Shoulder prosthesis according to one of claims 1 to 18, characterised in that the hemispherical element (19) has a plate which rests against the inner face of the acromion (5) of the shoulder blade (2), in which the glenoid part (8) is fixed.

**Patentansprüche**

1. Schulterprothese mit einem Oberarmstück (7), das in einem Oberarm (1), befestigt wird, um wenigstens dessen Kopf zu ersetzen, und das ein hohles rohrförmiges Element (9), das im Oberarm (1) im wesentlichen entlang dessen Längsachse befestigt werden soll, und einen zylindrischen Stab (10), dessen oberes Ende eine mit einer gelenkpfannenartigen Aufnahme zusammenwirkende Kugel (16) zum Bilden des Schultergelenks aufweist und dessen unteres Ende im vorgenannten rohrförmigen Element (9) angeordnet ist, wobei dieses und der Stab (10) gegeneinander verschiebbar sind, aufweist, wobei der zylindrische Stab (10) einen geraden Querschnitt aufweist, der im wesentlichen gleich dem der Aufnahme (11), die den Hohlraum im Inneren des rohrförmigen Elements (9) darstellt, ist, und wobei die gelenkpfannenartige Aufnahme aus einem gelenkpfannenartigen Stück (8), das ein zum Eingesetztwerden in eine im Schulterblatt (2) ausgebildete Aushöhlung bestimmtes Verankerungselement aufweist, und einem hohlen halbkugelförmigen Element (19), das mit der Kugel (16) des Oberarmstücks (7) zusammenwirkt, gebildet ist, dadurch gekennzeichnet, dass das Verankerungselement (18) einerseits an seinem oberen Teil einen Flügel (20), der gegenüber der Senkrechten um einen Winkel von etwa 30° nach vorn geneigt ist, der gegenüber der Seitenfläche des Verankerungselements zurückgesetzt ist und der dazu bestimmt ist, in die Einsetzbasis des korakoiden Knochenfortsatzes (4) des Schulterblatts (2) eingeführt zu werden, und andererseits an seinem unteren Teil eine Öffnung (22) zum Hindurchführen eines Mittels (23) zur Befestigung im Korpus des Schulterblatts (2) aufweist.

11

EP 0 278 807 B1

2. Schulterprothese nach Anspruch 1, dadurch gekennzeichnet, dass die äussere Wandung (12) des rohrförmigen Elements (7) an ihrem oberen Ende zu einem Kegelstumpf (13) aufgeweitet ist, dessen Grundflächen im wesentlichen senkrecht zur Achse des rohrförmigen Elements (7) verlaufen, wobei die obere Grundfläche (14) mit dem zum Entfernen des Kopfes (6) des Oberarms (1) angebrachten Quereinschnitt fluchtet.

3. Schulterprothese nach Anspruch 2, dadurch gekennzeichnet, dass die obere Grundfläche (14) des Kegelstumpfs (13) in Richtung der Innenseite des Oberarms (1) durch einen flachen Schnabel (15) verlängert ist.

4. Schulterprothese nach einem der Ansprüche 1 bis 3, dadurch gekennzeichnet, dass das untere Ende des rohrförmigen Elements (9) eine abgerundete Form aufweist.

5. Schulterprothese nach einem der Ansprüche 1 bis 4, dadurch gekennzeichnet, dass der gerade Querschnitt des Stabs (10) und jener der inneren Aufnahme (11) des rohrförmigen Elements (9) kreisförmig sind.

6. Schulterprothese nach einem der Ansprüche 1 bis 5, dadurch gekennzeichnet, dass die äussere Oberfläche des rohrförmigen Elements (9) horizontale Nuten zum oberen Ende desselben hin und vertikale Nuten aufweist.

7. Schulterprothese nach einem der Ansprüche 1 bis 6, dadurch gekennzeichnet, dass das rohrförmige Element (7) eine Länge zwischen 8 und 10 cm, eine Stärke der Wandung (12) zwischen 0,15 und 0,25 cm und einen Aussendurchmesser zwischen 10 und 12 mm aufweist, dass der Kegelstumpf (13) eine Höhe zwischen 2 und 2,5 cm und dessen obere Grundfläche (14) einen Durchmesser zwischen 20 und 30 mm aufweist und dass die innere Aufnahme (11) eine Tiefe zwischen 7 und 9 mm aufweist.

8. Schulterprothese nach einem der Ansprüche 1 bis 7, dadurch gekennzeichnet, dass der Stab (10) an seinem oberen Ende eine zu seiner Achse senkrechte Scheibe (17) aufweist, die in einem Abstand zur Kugel (16) angeordnet ist.

9. Schulterprothese nach Anspruch 8, dadurch gekennzeichnet, dass der Abstand zwischen dem unteren Ende (10a) des Stabs (10) und der Unterseite der Scheibe (17) höchstens gleich der Tiefe der inneren Aufnahme (11) des rohrförmigen Elements (9) ist.

10. Schulterprothese nach Anspruch 8 oder 9, dadurch gekennzeichnet, dass der Abstand zwischen der der Kugel (16) zugewandten Oberseite der Scheibe (17) und der Basis der Kugel (16) zwischen 12 und 15 mm beträgt.

11. Schulterprothese nach einem der Ansprüche 1 bis 10, dadurch gekennzeichnet, dass der Durchmesser der Kugel (16) zwischen 10 und 15 mm beträgt.

12. Schulterprothese nach einem der Ansprüche 1 bis 10, dadurch gekennzeichnet, dass der Durchmesser der Kugel (16) zwischen 35 und 50 mm beträgt.

13. Schulterprothese nach Anspruch 1, dadurch gekennzeichnet, dass das Verankerungselement (18) und das halbkugelförmige Element (19) zwei getrennte, durch ein Verbindungsmittel verbundene Elemente sind.

14. Schulterprothese nach Anspruch 1, dadurch gekennzeichnet, dass das Befestigungselement eine Schraube (23) ist.

15. Schulterprothese nach Anspruch 1, 13 oder 14, dadurch gekennzeichnet, dass das Verankerungselement (18) durch eine Masse (21) gebildet ist, deren horizontale Querschnitte im wesentlichen gleichschenklige Dreiecke sind.

16. Schulterprothese nach Anspruch 15, dadurch gekennzeichnet, dass der untere Teil (21b) der Masse (21) eine im wesentliche plane äussere Fläche (30) aufweist, die mit dem Knochen an der Gelenkpfan-

12

ne fluchtet und auf der ein quaderförmiger Block (25) aufsitzt, der mit Rückhaltemitteln (26), die mit dem Verbindungsmittel zusammenwirken, versehen ist, wobei ein Steg (31) an der Basis der äusseren Fläche (30) so vorgesehen ist, dass er dem Korpus des Schulterblatts (21) zugewandt ist, wobei der Steg (31) eine Öffnung zum Hindurchführen des Befestigungsmittels (23), das in den Korpus eindringt, aufweist, wobei der obere Teil (21a) der Masse (21) die Verbindung zwischen dem unteren Teil (21b) und dem Flügel (20) bildet, wobei die Breite der Masse (21) an ihrer Aussenseite, horizontal gemessen, in Höhe des Blocks (25) am grössten ist und sich zum Steg (31) und zum Flügel (20) hin verringert, wobei trotz allem eine Verengung zwischen dem oberen Teil (21a) und dem unteren Teil (21b) vorgesehen ist, und wobei der Flügel (20) gegenüber der äusseren Fläche (30) zurückgezogen angeordnet und mit dieser durch eine gegenüber der äusseren Fläche schräge Fläche (32) verbunden ist.

17. Schulterprothese nach Anspruch 16, dadurch gekennzeichnet, dass das mit dem Verbindungsmittel zusammenwirkende Mittel ein vertikaler Tunnel (26) ist, der den Block (25) vollständig durchdringt, wobei das Verbindungsmittel ein Stift ist.

18. Schulterprothese nach Anspruch 1, dadurch gekennzeichnet, dass das halbkugelförmige Element (19) eine halbkugelförmige Kuppel (27), in der die Kugel (16) des Oberarmstücks (7) gelagert ist, und einen Sitz (28), der den Block (25) des Verankerungselements (18) umgeben kann, aufweist.

19. Schulterprothese nach Anspruch 18, dadurch gekennzeichnet, dass die halbkugelförmige Kuppel (27) gegenüber der Kugel (16) des Oberarmstücks (7) eine leichte Rückhaltewirkung zeigt.

20. Schulterprothese nach einem der Ansprüche 1 bis 18, dadurch gekennzeichnet, dass das halbkugelförmige Element (19) eine Fläche aufweist, die auf der Innenfläche des spatenförmigen Knochenfortsatzes (5) des Schulterblatts (2), in dem das gelenkpfannenartige Stück (8) befestigt ist, aufliegt.

FIG. 4

FIG. 1

FIG. 6

FIG. 5

FIG. 2

FIG. 3

**FIG. 7**

**FIG. 8**

**FIG. 9**

**FIG. 10**

21

25    26

**FIG. 11**

40

14

41

13

15

12

9

11

**FIG. 12**

16 a

10 b

17

10

10 a

**FIG. 13**